# FASCICULE DE BREVET EUROPEEN

(11) **EP 1 093 808 B1**
(45) Date de publication et mention de la délivrance du brevet: **05.07.2006**
(21) Numéro de dépôt: 00402807.2
(22) Date de dépôt: 11.10.2000
(51) Int. Cl.: A61K 8/89, A61K 8/73, A61Q 19/10, A61Q 5/00, A61Q 5/12, A61Q 5/04, A61Q 5/10

(54) **Compositions cosmétiques contenant un copolymére vinyldiméthicone/diméthicone et un polymère cationique et leurs utilisations**
Kosmetische Zusammensetzungen enthaltend ein Vinyldimethicon/Dimethicon Copolymer und ein kationisches Polymer, und ihre Anwendungen
Cosmetic compositions containing a vinyldimethicone/dimethicone copolymer and a cationic polymer, and their uses

(30) Priorité: 20.10.1999 FR 9913098
(43) Date de publication de la demande: 25.04.2001
(73) Titulaire: L'ORÉAL, 75008 Paris (FR)
(72) Inventeur: Decoster, Sandrine, 95210 Saint Gratien (FR); Douin, Véronique, 75017 Paris (FR); Bailly, Virginie, 92110 Clichy (FR)
(74) Mandataire: Le Blainvaux Bellegarde, Françoise

(56) Documents cités:
- EP-A- 0 787 758
- EP-A- 0 874 017
- US-A- 4 839 166
- US-A- 4 996 059

## Description

La présente invention concerne de nouvelles compositions cosmétiques comprenant dans un milieu cosmétiquement acceptable au moins un polymère cationique et au moins un copolymère dimethicone à insaturation éthylénique/dimethicone.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de lissage (cheveu non homogène de la racine à la pointe).
En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

En résumé, il s'avère que les compositions cosmétiques actuelles contenant des polymères cationiques, ne donnent pas complètement satisfaction.

La demanderesse a maintenant découvert que l'association d'un copolymère siliconé défini ci-dessous de viscosité comprise entre 10⁶ et 100.10⁶ cP avec des polymères cationiques permet de remédier à ces inconvénients.

Ainsi, à la suite d'importantes recherches menées sur la question, il a maintenant été trouvé par la Demanderesse qu'en introduisant d'un copolymère siliconé défini ci-dessous de viscosité comprise entre 10⁶ et 100.10⁶ cP dans les compositions en particulier capillaires de l'art antérieur à base de polymères cationiques, il est possible de limiter, voire supprimer, les problèmes généralement liés à l'emploi de telles compositions, à savoir en particulier l'alourdissement (toucher chargé lors d'applications répétées), le manque de lissage et de douceur des cheveux, tout en conservant les autres propriétés cosmétiques avantageuses qui sont attachés aux compositions à base d'agents conditionneurs.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, au moins un copolymère siliconé défini ci-dessous de viscosité comprise entre 10⁶ et 100.10⁶ cP et au moins un polymère cationique.

Un autre objet de l'invention concerne l'utilisation d'un copolymère siliconé défini ci-dessous de viscosité comprise entre 10⁶ et 100.10⁶ cP dans, ou pour la fabrication d'une composition cosmétique comprenant un polymère cationique.

Les différents objets de l'invention vont maintenant être détaillés. L'ensemble des significations et définitions des composés utilisés dans la présente invention données ci-dessous sont valables pour l'ensemble des objets de l'invention.

Le copolymère siliconé résulte de la réaction d'addition, en présence d'un catalyseur, d'au moins :
- (a) un polysiloxane de formule (I) : 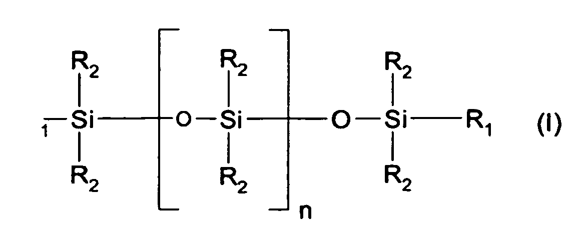 dans laquelle :
   R1 désigne un groupement susceptible de réagir par réaction d'addition de chaîne tel que par exemple un atome d'hydrogène, un groupe aliphatique en C2-C6 ayant une insaturation éthylénique notamment vinyl, allyl ou héxényl,
   Les groupements R2 de la formule (I) peuvent représenter notamment des groupements alkyle, alcényle cycloalkyle, aryle, alkylaryle ou hydroxyle, et peuvent comporter en outre, des groupements fonctionnels tels qu'éthers, amines, carboxyles, hydroxyles, thiols, esters, sulfonates, sulfates.
   Les groupements alkyle et alcényle ont par exemple 1 à 20 atomes de carbone ; les groupements cycloalkyle ont par exemple 5 ou 6 atomes de carbone ; les groupements aryle sont notamment des groupements phényle ; les groupements alkylaryle peuvent avoir de 7 à 20 atomes de carbone.
   Plus particulièrement R2 désigne méthyle.
   n est un entier tel que le polysiloxane de formule (I) ait de préférence une viscosité cinématique comprise entre 1 et 1.10⁶ mm²/s..n varie notamment de 5 à 5000.
- (b) et d'au moins un composé siliconé comprenant au moins un et au plus deux groupements susceptibles de réagir avec les groupements R1 du polysiloxane (a).
   l'un au moins des composés de type (a) ou (b) contient un groupe aliphatique en C2-C6 ayant une insaturation éthylénique.

Les composés de type (b) sont un autre polysiloxane de type (a) dans lequel les groupements R1 du polysiloxane (b) sont susceptibles de réagir avec les groupement R1 du polysiloxane (a).

De préférence, les copolymères siliconés sont notamment obtenus par réaction d'addition, en présence d'un catalyseur d'hydrosilylation (par exemple un catalyseur au platine), d'au moins :
- (a) un alpha,oméga-di vinyl polydiméthylsiloxane, et
- (b) d'un alpha,oméga-di hydrogéno polydiméthylsiloxane.

Le copolymère a généralement une viscosité dynamique, mesurée à la température d'environ 25°C et au taux de cisaillement de 0,01 Hz pour une contrainte de 1500 Pa, comprise entre 10⁶ et 100.10⁶ cP et de préférence comprise entre 5.10⁶ cP et 30.10⁶ cP.

Toutes les mesures de viscosités dynamiques données dans la présente demande ont été effectuées à une température d'environ 25°C, sur un Carri-Med CSL2-500.

La viscosité cinématique est par exemple mesurée à 25°C selon la norme ASTM 445 Appendice C.

Les copolymères siliconés selon l'invention sont essentiellement non réticulés.

Le copolymère siliconé présent dans la composition selon l'invention peut se présenter sous la forme d'émulsion aqueuse.
Par émulsion aqueuse, on entend une émulsion de type huile-dans-eau dans laquelle le copolymère siliconé est dispersé sous forme de particules ou de gouttelettes dans la phase aqueuse formant la phase continue de l'émulsion.
Cette émulsion peut être stabilisée par un système émulsionnant usuel.
Cette émulsion de silicone peut avoir une taille de gouttelettes ou de particules de silicone allant de 10 nm à 50 µm, et de préférence de 0,3 µm à 20 µm.
La taille des particules est mesurée par granulométrie laser.
Le système émulsionnant comprend des tensioactifs employés habituellement dans les émulsions de silicone. Ces tensioactifs peuvent être non-ioniques, cationiques, anioniques ou amphotères ou leurs mélanges tels que ceux décrits ci-dessous.

Le système émulsionnant représente de 0,5 % à 10 % en poids par rapport au poids total de l'émulsion.

La synthèse de ces émulsions de silicones est notamment décrite dans la demande EP-A-874017.

De telles émulsions sont notamment commercialisées sous la dénomination DC2-1997 cationic emulsion par la société DOW CORNING. Cette émulsion comprend un copolymère α,ω̅-divinyl diméthicone/α,ω̅-dihydrogénodiméthicone ayant une viscosité dynamique d'environ 15.10⁶ cP, un émulsionnant de type cationique tel que le chlorure de cétyltriméthylammonium, un stabilisant de type hydroxyéthylcellulose et de l'eau.

Le copolymère siliconé est utilisé de préférence en une quantité comprise entre 0,05 et 10% en poids par rapport au poids total de la composition. Plus préférentiellement, cette quantité est comprise entre 0,1 et 5% en poids par rapport au poids total de la composition.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 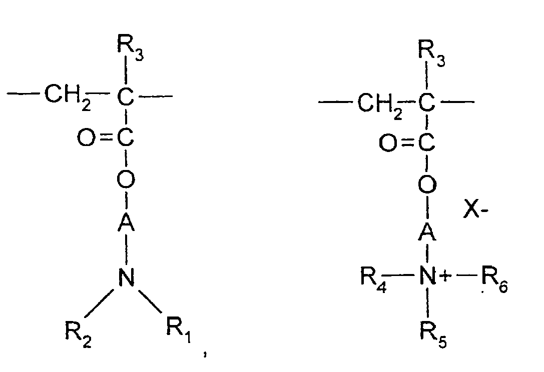 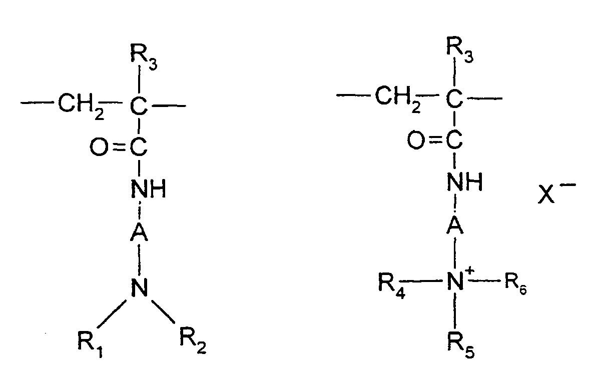 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.
      Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
      Ainsi, parmi ces copolymères de la famille (1), on peut citer :
      - les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
      - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
      - le copolymère d'acrylamide et de méthosulfate de méthacryloyloxyéthyltriméthylammonium vendu sous la dénomination RETEN par ia société HERCULES,
      - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
      - les terpolymères méthacrylate de diméthyl amino éthyle/ vinylcaprolactame/ vinylpyrrolidone tel que le produit vendu sous la dénomination GAFFIX VC 713 par la société ISP,
      - les copolymère vinylpyrrolidone / méthacrylamidopropyl dimethylamine commercialisés notamment sous la dénomination STYLEZE CC 10 par ISP.
      - et les copolymères vinylpyrrolidone / méthacrylamide de diméthylaminopropyle quaternisé tel que le produit vendu sous la dénomination "GAFQUAT HS 100" par la société ISP.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) les cyclopolymères d'alkyl diallyl amine ou de dialkyl diallyl ammonium tels que les homopolymères ou copolymères comportant comme constituant principal de la chaîne des motifs répondant aux formules (VI) ou (VI') : 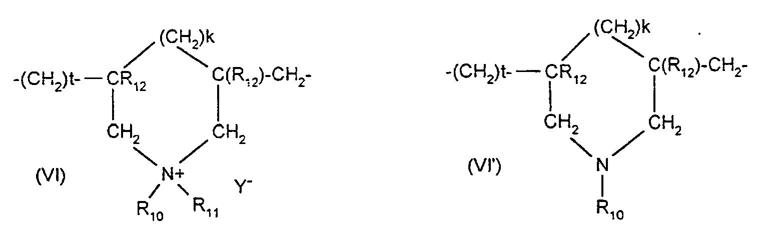 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faibles masses moléculaires moyenne en poids) et les copolymères de chlorure de diallyldiméthylammonium et d'acrylamide commercialisés sous la dénomination "MERQUAT 550".
(10) le polymère de diammonium quaternaire contenant des motifs récurants répondant à la formule : 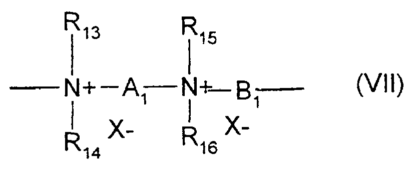 formule (VII) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ;
      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
      On peut utiliser plus particulièrement les polymères qui sont constitués de motifs récurrents répondant à la formule : 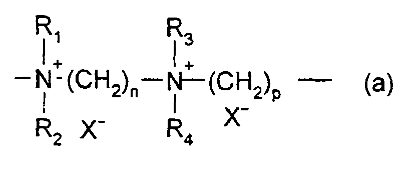 dans laquelle R₁, R₂, R₃ et R_{4,} identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
      Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(11) les polymères de polyammonium quaternaires constitués de motifs de formule (VIII): 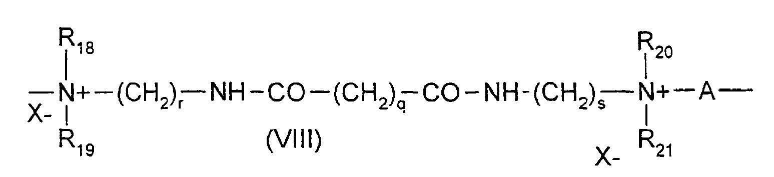 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogènure,
   A désigne un radical d'un dihalogénure ou représente de préférence -CH₂-CH₂-O-CH₂-CH₂-.
   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
(12) Les polymères quaternaires de vinylpyrrolidone et de vinylimidazole tels que par exemple les produits commercialisés sous les dénominations Luviquat® FC 905, FC 550 et FC 370 par la société B.A.S.F.
(13) Les polyamines comme le Polyquart® H vendu par HENKEL, référencé sous le nom de « POLYETHYLENEGLYCOL (15) TALLOW POLYAMINE » dans le dictionnaire CTFA.
(14) Les polymères réticulés de sels de (méth)acryloyloxyalkyl(C₁-C₄) trialkyl(C₁₋C₄)ammonium tels que les polymères obtenus par homopolymérisation du diméthylaminoéthylméthacrylate quaternisé par le chlorure de méthyle, ou par copolymérisation en particulier de l'acrylamide avec le diméthylaminoéthylméthacrylate quaternisé par un halogénure (chlorure) de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société ALLIED COLLOIDS.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société CALGON, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium et leurs mélanges.

Selon l'invention, le ou les polymères cationiques peuvent représenter de 0,001 % à 20 % en poids, de préférence de 0,01 % à 10% en poids et plus particulièrement de 0,1 à 3% en poids par rapport au poids total de la composition finale.

Les compositions de l'invention peuvent contenir outre au moins un agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈₋C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉-COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

Encore plus préférentiellement les compositions selon l'invention peuvent contenir en outre au moins un tensioactif cationique.
Les tensioactifs cationiques peuvent être choisis parmi :
A) les sels d'ammonium quaternaires de la formule générale (IV) suivante : 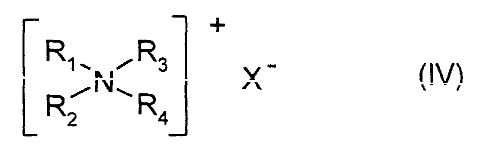 dans laquelle X est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
   et
   i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R4 désigne un radical alkyle, linéaire ou ramifié, comportant de 16 à 30 atomes de carbone.
      De préférence le tensioactif cationique est un sel (par exemple chlorure) de béhényl triméthyl ammonium.
   ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
      R3 et R4, identiques ou différents, désignent un radical alkyle, linéaire ou ramifié, comportant de 12 à 30 atomes de carbone, ledit radical comprenant au moins une fonction ester ou amide.
      R3 et R4 sont notamment choisis parmi les radicaux alkyl(C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
B) - les sels d'ammonium quaternaire de l'imidazolinium, comme par exemple celui de formule (V) suivante : 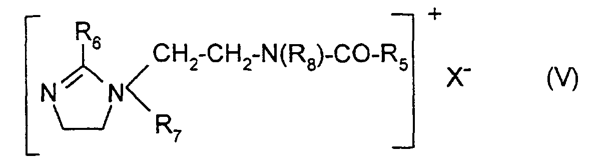
dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) - les sels de diammonium quaternaire de formule (VI) : 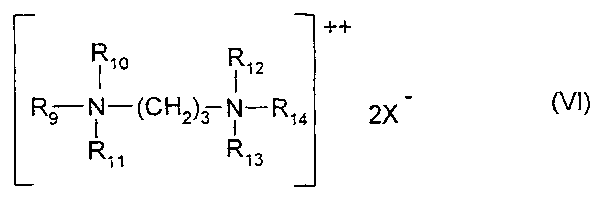 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire contenant au moins une fonction ester de formule (VII) suivante : 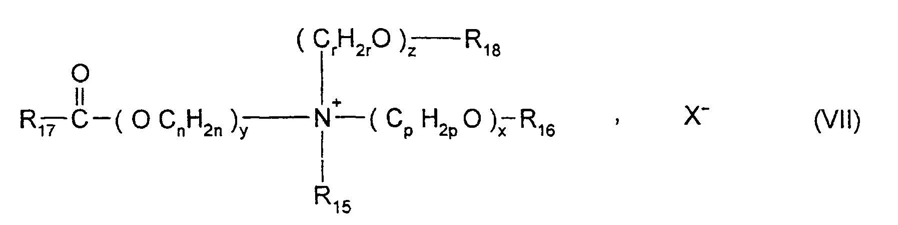 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
      - le radical 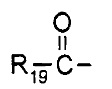
      - les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical 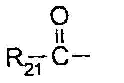
      - les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
   sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (VII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 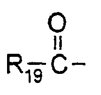
   - les radicaux méthyle, éthyle ou hydrocarbonés en C₁₄-C₂₂
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
   - le radical 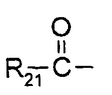
   - l'atome d'hydrogène ;

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Le tensioactif cationique est généralement présent dans des concentrations allant de 0,1 à 10% en poids par rapport au poids total de la composition et de préférence de 0,5 à 7 % en poids et plus préférentiellement entre 1 et 5% en poids.

La composition de l'invention peut également contenir au moins un additif choisi parmi les épaississants, les parfums, les agents nacrants, les conservateurs, les filtres solaires siliconés ou non, les vitamines, les provitamines, les polymères amphotères, anioniques ou non ioniques, les protéines, les hydrolysats de protéine, l'acide méthyl-18 eicosanoique, les hydroxyacides, le panthénol, les silicones volatiles ou non volatiles, cycliques ou linéaires ou réticulés, modifiées ou non, les céramides, les pseudocéramides, les huiles végétales, animales, minérales ou de synthèse et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention peuvent être des compositions d'après-shampooing à rincer ou non.
Les compositions selon l'invention peuvent être également des compositions détergentes telles que des shampooings, des gels-douche, des bains moussants et peuvent être également des démaquillants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères et non ioniques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

Dans les exemples, les noms commerciaux ont les définitions suivantes :

### EXEMPLE 1

On a réalisé un après-shampooing conforme à l'invention de composition suivante :
- Mélange de mono-, di- et tristéarate de glycérol 1 g
- Glycérol 0,5 g
- Polyquaternium-11 en solution aqueuse à 20% de matière active (MA) (GAFQUAT 755 de ISP 0,5 gMA
- Polyquaternium-30 en solution hydroalcoolique à 22% de MA (MEXOMERE PX de CHIMEX) 0,55 gMA
- Chlorure de béhényl triméthyl ammonium (GENAMIN KDMP de CLARIANT) 1,45 g MA
- Emulsion cationique à 67% MA de copolymère polydiméthylsiloxane à groupements alpha-oméga vinyle / polydiméthylsiloxane à groupements alpha-oméga hydrogéno (DC-1997 de DOW CORNING) 0,8 gMA
- Mélange d'alcool cétylique et d'alcool stéarylique (50/50 en poids) 4 g
- Parfum, conservateurs qs
- Eau qsp 100 g

On applique cette composition sur des cheveux lavés et essorés. On laisse pauser pendant 2 minutes, puis on rince à l'eau.
Les cheveux traités avec cet après-shampooing sont doux, lisses et se démêlent facilement.

### EXEMPLE 2

On a réalisé un après-shampooing conforme à l'invention de composition suivante :
- Emulsion cationique à 67% MA de copolymère polydiméthylsiloxane à groupements alpha-oméga vinyle / polydiméthylsiloxane à groupements alpha-oméga hydrogéno (DC-1997 de DOW CORNING) 5 gMA
- Copolymère SMDI / polyéthylène glycol / terminaisons alkyle (méthyl/C18) à 15% dans une matrice maltodextrine / eau (ACULYN 46 de ROHM HAAS) 0,45 gMA
- Homopolymère chlorure de méthacrylate d'éthyl triméthyl ammonium réticulé en émulsion inverse à 50% dans de l'huile minérale (SALCARE SC 95 de CIBA GEIGY) 0,55 gMA
- Mélange d'alcool cétylique et d'alcool stéarylique (50/50 en poids) 6 g
- Parfum, conservateurs qs
- Eau qsp 100 g

### EXEMPLE 3

On a réalisé un shampooing conforme à l'invention de composition suivante :
- Emulsion cationique à 67% MA de copolymère polydiméthylsiloxane à groupements alpha-oméga vinyle / polydiméthylsiloxane à groupements alpha-oméga hydrogéno (DC-1997 de DOW CORNING) 1,95 gMA
- Lauryléther sulfate de sodium oxyéthyléné à 2,2 moles d'oxyde d'éthylène en solution aqueuse à 70% de MA 15,3 gMA
- Tensioactif dérivé d'imidazoline en solution aqueuse à 38% de matière active(MIRANOL C2M conc de RHODIA CHIMIE) 3,05 g MA
- Gomme de guar modifiée par du chlorure de 2,3-époxypropyl triméthylammonium (JAGUAR C13 S de RHODIA CHIMIE) 0,2 g
- Mélange d'alcool stéarylique (10%) et de distéaryléther (90%) 1,5 g
- Mélange d'alcools linéaires (C18/C20/C22) (NAFOL 1822 C de CONDEA) 1,5 g
- Alcool laurique oxyéthyléné 2,5 moles d'oxyde d'éthylène 0,75 g
- Monoisopropanolamide d'acide de coprah 0,4 g
- Acide polyacrylique réticulé 0,2 g
- Vitamine B3, Vitamine B6 qs
- Extrait de fruits qs
- Conservateurs, parfum qs
- Acide citrique, 1H2O qs pH 7,5
- Eau déminéralisée qsp 100 g

## Revendications

1. Composition cosmétique, **caractérisée par le fait qu'**elle comprend, dans un milieu cosmétiquement acceptable, au moins un polymère cationique et au moins un copolymère siliconé de viscosité comprise entre 10⁶ et 100.10⁶ cP résultant de la réaction d'addition, en présence d'un catalyseur, d'au moins :
- (a) un polysiloxane de formule (I) ; 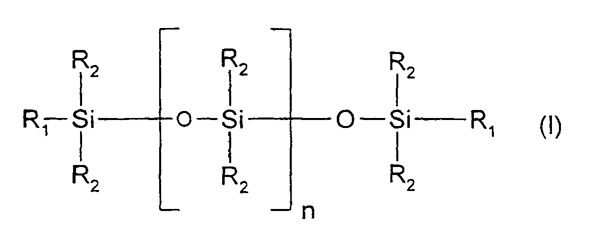
dans laquelle :
R1 désigne un groupement susceptible de réagir par réaction d'addition de chaîne tel que par exemple un atome d'hydrogène, un groupe aliphatique en C2-C6 ayant une insaturation éthylénique, notamment vinyl, allyl ou héxényl,
les groupements R2 de la formule (I) représentent des groupements alkyle ayant de 1 à 20 atomes de carbone, alcényle ayant de 1 à 20 atomes de carbone, cycloalkyle ayant 5 ou 6 atomes de carbone, aryle, alkylaryle ayant de 7 à 20 atomes de carbone ou hydroxyle, et peuvent comporter en outre, des groupements fonctionnels tels qu'éthers, amines, carboxyles, hydroxyles, thiols, esters, sulfonates, sulfates,
n est un entier tel que le polysiloxane de formule (I) ait de préférence une viscosité cinématique comprise entre 1 et 1.10⁶ mm²/s.
- (b) et d'au moins un composé siliconé comprenant au moins un et au plus deux groupements susceptibles de réagir avec les groupements R1 du polysiloxane (a), l'un au moins des composés de type (a) ou (b) contient un groupe aliphatique en C2-C6 ayant une insaturation éthylénique.

2. Composition selon la revendication 1, **caractérisée par le fait que** R2 désigne méthyle.

3. Composition selon l'une des revendications 1 ou 2, **caractérisée par le fait que** le composé de type (b) est un autre polysiloxane de type (a) dans lequel les groupements R1 du polysiloxane (b) sont susceptibles de réagir avec les groupement R1 du polysiloxane (a).

4. Composition selon l'une quelconque des revendications 1 à 3, **caractérisée par le fait que** le copolymère siliconé est obtenu par réaction d'addition, en présence d'un catalyseur d'hydrosilylation, d'au moins :
- (a) un alpha,oméga-di vinyl polydiméthylsiloxane, et
- (b) d'un alpha,oméga-di hydrogéno polydiméthylsiloxane.

5. Composition selon l'une quelconque des revendications 1 à 4, **caractérisée par le fait que** le copolymère siliconé est sous la forme d'émulsion aqueuse.

6. Composition selon l'une quelconque des revendications 1 à 5, **caractérisée par le fait que** le copolymère siliconé est présent à une concentration comprise entre 0,05 et 10% en poids par rapport au poids total de la composition.

7. Composition selon l'une quelconque des revendications précédentes, **caractérisée par le fait que** ledit polymère cationique est choisi parmi :
(1) les homopolymères ou copolymères dérivés d'esters ou d'amides acryliques ou méthacryliques et comportant au moins un des motifs de formules suivantes: 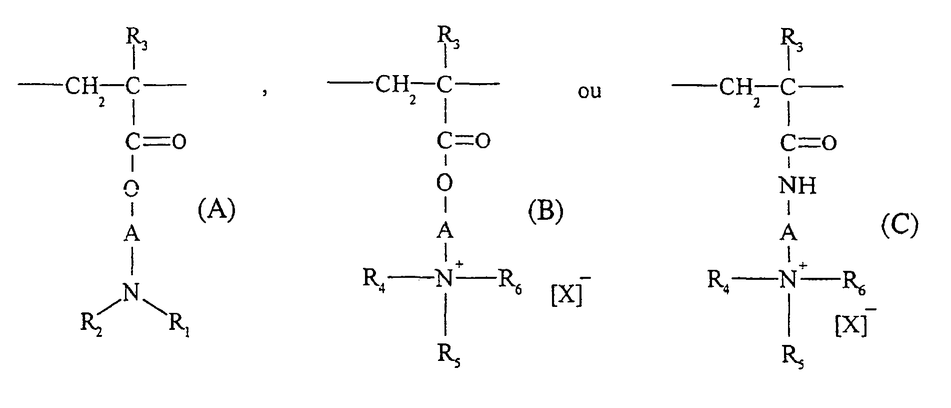 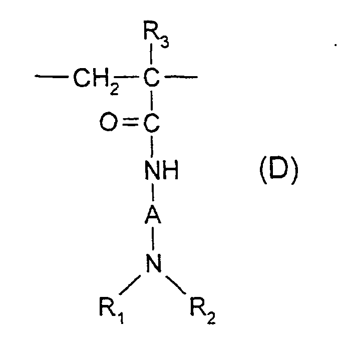 dans lesquelles:
R₃, identiques ou différents, désignent un atome d'hydrogène ou un radical CH₃;
A, identiques ou différents, représentent un groupe alkyle, linéaire ou ramifié, de 1 à 6 atomes de carbone ou un groupe hydroxyalkyle de 1 à 4 atomes de carbone ;
R₄, R₅, R₆, identiques ou différents, représentent un groupe alkyle ayant de 1 à 18 atomes de carbone ou un radical benzyle;
R₁ et R₂, identiques ou différents, représentent hydrogène ou un groupe alkyle ayant de 1 à 6 atomes de carbone;
X désigne un anion dérivé d'un acide minéral ou organique.
(2) Les dérivés d'éthers de cellulose comportant des groupements ammonium quaternaires,
(3) Les dérivés de cellulose cationiques tels que les copolymères de cellulose ou les dérivés de cellulose greffés avec un monomère hydrosoluble d'ammonium quaternaire,
(4) Les polysaccharides cationiques,
(5) les polymères constitués de motifs pipérazinyle et de radicaux divalents alkylène ou hydroxyalkylène à chaînes droites ou ramifiées, éventueiiement interrompues par des atomes d'oxygène, de soufre, d'azote ou par des cycles aromatiques ou hétérocycliques, ainsi que les produits d'oxydation et/ou de quaternisation de ces polymères,
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées.
(7) les dérivés de polyaminoamides résultant de la condensation de polyalcoylènes polyamines avec des acides polycarboxyliques suivie d'une alcoylation par des agents bifonctionnels,
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone,
(9) les cyclopolymères d'alkyl diallyl amine ou de dialkyl diallyl ammonium
(10) le polymère de diammonium quaternaire contenant des motifs récurrents répondant à la formule : 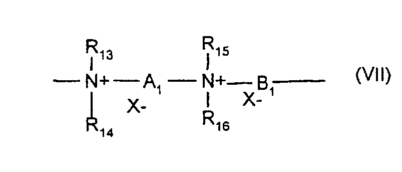 formule (VII) dans laquelle:
R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :
-(CH2-CH2-O)x-CH2-CH2-
-[CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent -CH2-CH2-S-S-CH2-CH2- ;
d) un groupement uréylène de formule : -NH-CO-NH- ;
De préférence, X⁻ est un anion.
(11) les polymères de polyammonium quaternaires constitués de motifs de formule (VIII): 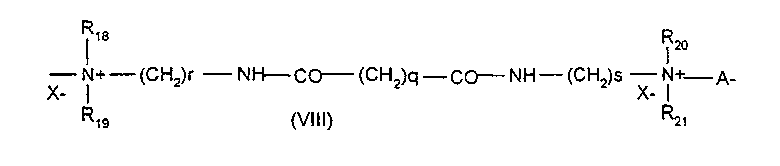 formule dans laquelle :
R18, R19, R20 et R21, identiques ou différents, représentent un atome d'hydrogène ou un radical méthyle, éthyle, propyle, β-hydroxyéthyle, β-hydroxypropyle ou -CH2CH2(OCH2CH2)pOH,
où p est égal à 0 ou à un nombre entier compris entre 1 et 6, sous réserve que R18, R19, R20 et R21 ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence -CH2-CH2-O-CH2-CH2-.
(12) Les polymères quaternaires de vinylpyrrolidone et de vinylimidazole,
(13) Les polyamines référencées sous le nom de « POLYETHYLENEGLYCOL (15) TALLOW POLYAMINE » dans le dictionnaire CTFA.
(14) Les polymères réticulés de sels de méthacryloyloxyalkyl(C₁-C₄) trialkyl(C₁-C₄)ammonium,
(15) des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

8. Composition selon la revendication précédente, **caractérisée par le fait que** ledit polymère cationique est choisi parmi les dérivés d'éther de cellulose quaternaires, les cyclopolymères, les polysaccharides cationiques, les copolymères vinylpyrrolidone / méthacrylamidopropyl dimethylamine, les homopolymères ou copolymères réticulés de sels de méthacryloyloxyalkyl(C₁-C₄) trialkyl(C₁-C₄)ammonium et leurs mélanges.

9. Composition selon l'une quelconque des revendications précédentes, **caractérisée en ce que** le polymère cationique est présent à une concentration comprise entre 0,001 % et 20 % en poids par rapport au poids total de la composition, de préférence entre 0,01 % et 10 % en poids.

10. Composition selon l'une quelconque des revendications précédentes, **caractérisée par le fait qu'**elle comprend en outre au moins un agent tensioactif choisi parmi les tensioactifs anioniques, non ioniques, amphotères, cationiques et leurs mélanges.

11. Composition selon la revendication 10, **caractérisée par le fait que** le ou les agents tensioactifs sont présents à une concentration comprise entre 0,1% et 60% en poids, de préférence entre 3% et 40% en poids, et encore plus préférentiellement entre 5% et 30% en poids, par rapport au poids total de la composition.

12. Composition selon l'une quelconque des revendications précédentes, **caractérisée par le fait qu'**elle comprend en outre au moins un agent tensioactif cationique.

13. Composition selon la revendication précédente, **caractérisée par le fait que** le tensioactif cationique est présent dans des concentrations allant de 0,1 à 10% en poids par rapport au poids total de la composition et de préférence de 0,5 à 7 % en poids et plus préférentiellement entre 1 et 5% en poids.

14. Composition selon l'une quelconque des revendications 1 à 11, **caractérisée par** le fait la composition contenir au moins un additif choisi parmi les épaississants, les parfums, les agents nacrants, les conservateurs, les filtres solaires siliconés ou non, les vitamines, les provitamines, les polymères amphotères, anioniques ou non ioniques, les protéines, les hydrolysats de protéine, l'acide méthyl-18 eicosanoique, les hydroxyacides, le panthénol, les silicones volatiles ou non volatiles, cycliques ou linéaires ou réticulés, modifiées ou non, les céramides, les pseudocéramides, les huiles végétales, animales, minérales ou de synthèse.

15. Composition selon l'une quelconque des revendications précédentes, **caractérisée par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour le corps.

16. Utilisation d'une composition telle que définie dans l'une quelconque des revendications précédentes pour le lavage ou pour le soin des matières kératiniques.

17. Procédé de traitement des matières kératiniques, telles que les cheveux, **caractérisé en ce qu'**il consiste à appliquer sur lesdites matières une composition cosmétique selon l'une des revendications 1 à 15, puis à effectuer éventuellement un rinçage à l'eau.

18. Utilisation d'un copolymère siliconé tel que défini à l'une des revendications 1 à 5 dans, ou pour la fabrication d'une composition cosmétique comprenant un polymère cationique.

## Claims

1. Cosmetic composition, **characterized in that** it comprises, in a cosmetically acceptable medium, at least one cationic polymer and at least one silicone copolymer with a viscosity of between 10⁶ and 100 × 10⁶ cP, resulting from the addition reaction, in the presence of a catalyst, of at least:
- (a) one polysiloxane of formula (I): 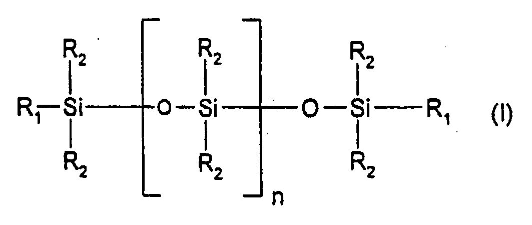
in which:
R₁ denotes a group which can react by chain addition reaction such as, for example, a hydrogen atom or a C₂-C₆ aliphatic group containing an ethylenic unsaturation, in particular vinyl, allyl or hexenyl; the groups R₂ in formula (I) represent alkyl groups containing from 1 to 20 carbon atoms, alkenyl groups containing from 1 to 20 carbon atoms, cycloalkyl groups containing from 5 to 6 carbon atoms, aryl groups, alkylaryl groups containing from 7 to 20 carbon atoms or hydroxyl groups and can also comprise functional groups such as ethers, amines, carboxyls, hydroxyls, thiols, esters, sulphonates or sulphates.
n is an integer such that the polysiloxane of formula (I) preferably has a kinetic viscosity of between 1 and 1 × 10⁶ mm²/s.
- (b) and of at least one silicone compound comprising at least one and not more than two groups capable of reacting with the groups R₁ of the polysiloxane (a), at least one of the compounds of type (a) or (b) contains an aliphatic C₂-C₆ group containing an ethylenic unsaturation.

2. Composition according to Claim 1, **characterized in that** R₂ denotes methyl.

3. Composition according to either of Claims 1 and 2, **characterized in that** the compound of type (b) is another polysiloxane of type (a) in which the groups R₁ of the polysiloxane (b) can react with the groups R₁ of the polysiloxane (a).

4. Composition according to any one of Claims 1 to 3, **characterized in that** the silicone copolymer is obtained by addition reaction, in the presence of a hydrosilylation catalyst, of at least:
- (a) one α,ω-divinylpolydimethylsiloxane, and
- (b) one α,ω-dihydrogenopolydimethylsiloxane.

5. Composition according to any one of Claims 1 to 4, **characterized in that** the silicone copolymer is in the form of an aqueous emulsion.

6. Composition according to any one of Claims 1 to 5, **characterized in that** the silicone copolymer is present at a concentration of between 0.05% and 10% by weight relative to the total weight of the composition.

7. Composition according to any one of the preceding claims, **characterized in that** the said cationic polymer is chosen from:
(1) homopolymers or copolymers derived from acrylic or methacrylic esters or amides and comprising at least one of the units of the following formulae: 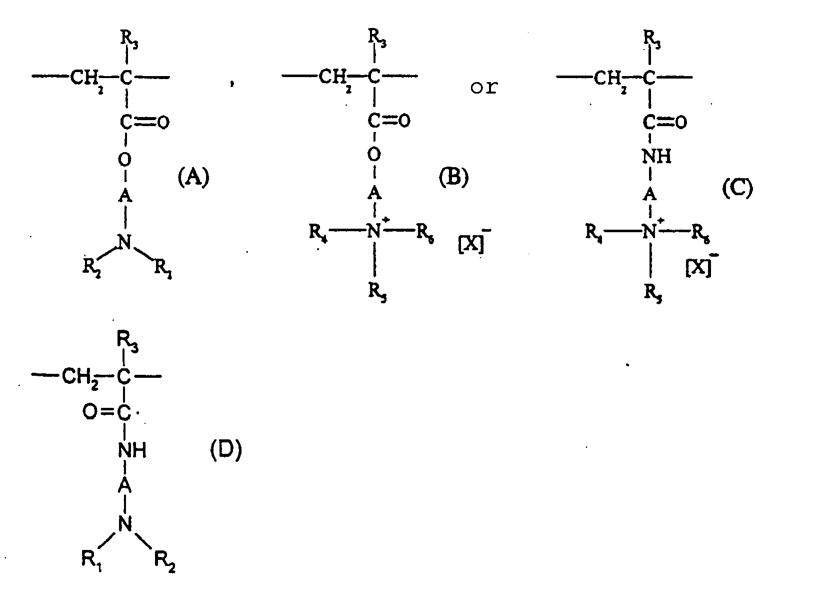 in which:
R₃, which may be identical or different, denote a hydrogen atom or a CH₃ radical;
A, which may be identical or different, represent a linear or branched alkyl group of 1 to 6 carbon atoms, or a hydroxyalkyl group of 1 to 4 carbon atoms;
R₄, R₅ and R₆, which may be identical or different, represent an alkyl group containing from 1 to 18 carbon atoms or a benzyl radical;
R₁ and R₂, which may be identical or different, represent hydrogen or an alkyl group containing from 1 to 6 carbon atoms;
X denotes an anion derived from an inorganic or organic acid,
(2) cellulose ether derivatives comprising quaternary ammonium groups,
(3) cationic cellulose derivatives such as cellulose copolymers or cellulose derivatives grafted with a water-soluble quaternary ammonium monomer,
(4) cationic polysaccharides,
(5) polymers consisting of piperazinyl units and of divalent alkylene or hydroxyalkylene radicals containing straight or branched chains, optionally interrupted by oxygen, sulphur or nitrogen atoms or by aromatic or heterocyclic rings, as well as the oxidation and/or quaternization products of these polymers,
(6) water-soluble polyamino amides prepared in particular by polycondensation of an acidic compound with a polyamine; these polyamino amides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent is used in proportions ranging from 0.025 to 0.35 mol per amine group of the polyamino amide; these polyamino amides can be alkylated or, if they contain one or more tertiary amine functions, they can be quaternized,
(7) the polyamino amide derivatives resulting from the condensation of polyalkylene polyamines with polycarboxylic acids followed by alkylation with difunctional agents,
(8) the polymers obtained by reaction of a polyalkylene polyamine containing two primary amine groups and at least one secondary amine group with a dicarboxylic acid chosen from diglycolic acid and saturated aliphatic dicarboxylic acids having from 3 to 8 carbon atoms,
(9) alkyldiallylamine or dialkyldiallylammonium cyclopolymers,
(10) the quaternary diammonium polymer containing repeating units corresponding to the formula: 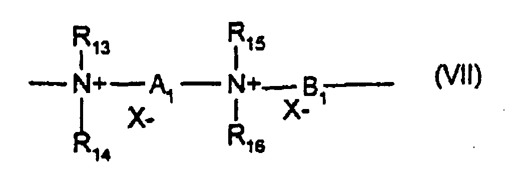 in which formula (VII):
R₁₃, R₁₄, R₁₅ and R₁₆, which may be identical or different, represent aliphatic, alicyclic or arylaliphatic radicals containing from 1 to 20 carbon atoms or lower hydroxyalkylaliphatic radicals, or alternatively R₁₃, R₁₄, R₁₅ and R₁₆, together or separately, constitute, with the nitrogen atoms to which they are attached, heterocycles optionally containing a second heteroatom other than nitrogen, or alternatively R₁₃, R₁₄, R₁₅ and R₁₆ represent a linear or branched C₁-C₆ alkyl radical substituted with a nitrile, ester, acyl or amide group or a group -CO-O-R₁₇-D or -CO-NH-R₁₇-D where R₁₇ is an alkylene and D is a quaternary ammonium group;
A₁ and B₁ represent polymethylene groups containing from 2 to 20 carbon atoms which may be linear or branched, saturated or unsaturated, and which may contain, linked to or intercalated in the main chain, one or more aromatic rings or one or more oxygen or sulphur atoms or sulphoxide, sulphone, disulphide, amino, alkylamino, hydroxyl, quaternary ammonium, ureido, amide or ester groups, and
X⁻ denotes an anion derived from an inorganic or organic acid;
A₁, R₁₃ and R₁₅ can form, with the two nitrogen atoms to which they are attached, a piperazine ring; in addition, if A₁ denotes a linear or branched, saturated or unsaturated alkylene or hydroxyalkylene radical, B₁ can also denote a group (CH₂)ₙ-CO-D-OC-(CH₂)ₙ-
in which D denotes:
a) a glycol residue of formula: -O-Z-O-, where Z denotes a linear or branched hydrocarbon radical or a group corresponding to one of the following formulae:
-(CH₂-CH₂-O)ₓ-CH₂-CH₂-
-[CH₂-CH (CH₃)-O]_{y}-CH₂-CH(CH₃)-
where x and y denote an integer from 1 to 4, representing a defined and unique degree of polymerization or any number from 1 to 4 representing an average degree of polymerization;
b) a bis-secondary diamine residue such as a piperazine derivative;
c) a bis-primary diamine residue of formula: -NH-Y-NH-, where Y denotes a linear or branched hydrocarbon radical, or alternatively the divalent radical
-CH₂-CH₂-S-S-CH₂-CH₂-;
d) a ureylene group of formula: -NH-CO-NH-;
X⁻ is preferably an anion,
(11) polyquaternary ammonium polymers consisting of units of formula (VIII): 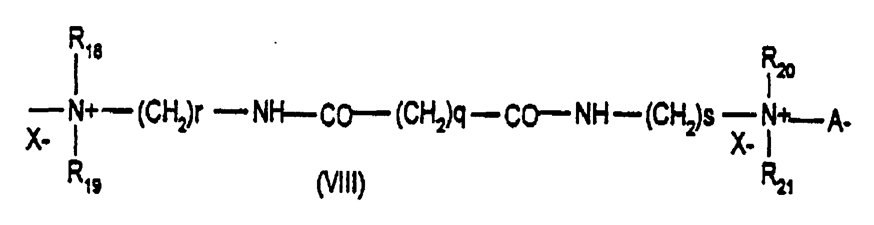 in which formula:
R₁₈, R₁₉, R₂₀ and R₂₁, which may be identical or different, represent a hydrogen atom or a methyl, ethyl, propyl, β-hydroxyethyl, β-hydroxypropyl or -CH₂CH₂(OCH₂CH₂)ₚOH radical,
where p is equal to 0 or to an integer between 1 and 6, with the proviso that R₁₈, R₁₉, R₂₀ and R₂₁ do not simultaneously represent a hydrogen atom,
r and s, which may be identical or different, are integers between 1 and 6,
q is equal to 0 or to an integer between 1 and 34,
X denotes a halogen atom,
A denotes a dihalide radical or preferably represents -CH₂-CH₂-O-CH₂-CH₂-,
(12) quaternary polymers of vinylpyrrolidone and of vinylimidazole,
(13) the polyamines referred to under the name "Polyethylene glycol (15) Tallow Polyamine" in the CTFA dictionary,
(14) crosslinked polymers of methacryloyloxy(C₁-C₄)-alkyltri(C₁-C₄) alkylammonium salts,
(15) polyalkyleneimines, in particular polyethyleneimines, polymers containing vinylpyridine or vinylpyridinium units, condensates of polyamines and of epichlorohydrin, polyquaternary ureylenes and chitin derivatives.

8. Composition according to the preceding claim, **characterized in that** the said cationic polymer is chosen from quaternary cellulose ether derivatives, cyclopolymers, cationic polysaccharides, vinylpyrrolidone/methacrylamidopropyldimethylamine copolymers, crosslinked homopolymers or copolymers of methacryloyloxy (C₁-C₄) alkyltri (C₁-C₄) alkylammonium salts, and mixtures thereof.

9. Composition according to any one of the preceding claims, **characterized in that** the cationic polymer is present at a concentration of between 0.001% and 20% by weight relative to the total weight of the composition, preferably between 0.01% and 10% by weight.

10. Composition according to any one of the preceding claims, **characterized in that** it also comprises at least one surfactant chosen from anionic, nonionic, amphoteric and cationic surfactants, and mixtures thereof.

11. Composition according to Claim 10, **characterized in that** the surfactant(s) is (are) present at a concentration of between 0.1% and 60% by weight, preferably between 3% and 40% by weight and even more preferably between 5% and 30% by weight, relative to the total weight of the composition.

12. Composition according to any one of the preceding claims, **characterized in that** it also comprises at least one cationic surfactant.

13. Composition according to the preceding claim, **characterized in that** the cationic surfactant is present in concentrations ranging from 0.1% to 10% by weight relative to the total weight of the composition, and preferably from 0.5% to 7% by weight and more preferably between 1% and 5% by weight.

14. Composition according to any one of Claims 1 to 11, **characterized in that** it contains at least one additive chosen from thickeners, fragrances, nacreous agents, preserving agents, silicone or non-silicone sunscreens, vitamins, provitamins, amphoteric, anionic or nonionic polymers, proteins, protein hydrolysates, 18-methyleicosanoic acid, hydroxy acids, panthenol, volatile or non-volatile, cyclic or linear or crosslinked, modified or non-modified silicones, ceramides, pseudoceramides, plant, animal, mineral or synthetic oils.

15. Composition according to any one of the preceding claims, **characterized in that** it is in the form of a shampoo, a conditioner, a composition for permanent-waving, straightening, dyeing or bleaching the hair, a rinse-out composition to be applied between the two steps of a permanent-waving or hair-straightening operation, or washing compositions for the body.

16. Use of a composition as defined in any one of the preceding claims, for washing or caring for keratin materials.

17. Process for treating keratin materials, such as the hair, **characterized in that** it consists in applying a cosmetic composition according to one of Claims 1 to 15, to the said keratin materials, and then in optionally rinsing it out with water.

18. Use of a silicone copolymer as defined in one of Claims 1 to 5, in, or for the manufacture of, a cosmetic composition comprising a cationic polymer.

## Patentansprüche

1. Kosmetische Zusammensetzung, **dadurch gekennzeichnet, dass** sie in einem kosmetisch akzeptablen Medium mindestens ein kationisches Polymer und mindestens ein Siliconcopolymer mit einer Viskosität von 10⁶ bis 100·10⁶ cP enthält, das in Gegenwart eines Katalysators bei der Addition von zumindest den folgenden Verbindungen gebildet wird:
(a) einem Polysiloxan der Formel (I): 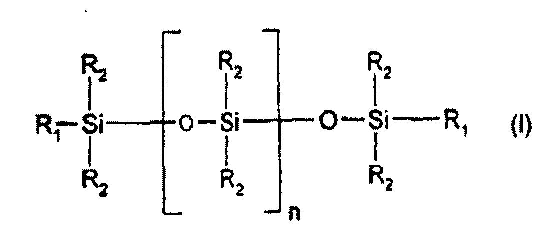 worin bedeuten:
R1 eine Gruppe, die durch Kettenaddition reagieren kann, wie beispielsweise ein Wasserstoffatom, eine aliphatische, ethylenisch ungesättigte Gruppe mit 2 bis 6 Kohlenstoffatomen, besonders Vinyl, Allyl oder Hexenyl,
wobei die Gruppen R2 der Formel (I) Alkylgruppen mit 1 bis 20 Kohlenstoffatomen, Alkenylgruppen mit 1 bis 20 Kohlenstoffatomen, Cycloalkylgruppen mit 5 oder 6 Kohlenstoff atomen, Arylgruppen, Alkylarylgruppen mit 7 bis 20 Kohlenstoffatomen oder Hydroxy bedeuten und ferner funktionelle Gruppen enthalten können, wie beispielsweise Ether, Amine, Carboxygruppen, Hydroxygruppen, Thiole, Ester, Sulfonate, Sulfate,
n eine ganze Zahl, die so gewählt ist, dass das Polysiloxan der Formel (I) vorzugsweise eine kinematische Viskosität von 1 bis 1.10⁶ mm²/s aufweist;
(b) und mindestens einer siliconierten Verbindung, die mindestens eine und höchstens zwei Gruppen aufweist, die mit den Gruppen R1 des Polysiloxans (a) reagieren können,
wobei mindestens eine der Verbindungen vom Typ (a) oder (b) eine aliphatische Gruppe mit 2 bis 6 Kohlenstoffatomen enthält, die ethylenisch ungesättigt ist.

2. Zusammensetzung nach Anspruch 1, **dadurch gekennzeichnet, dass** R2 Methyl bedeutet.

3. Zusammensetzung nach einem der Ansprüche 1 oder 2, **dadurch gekennzeichnet, dass** die Verbindung vom Typ (b) ein anderes Polysiloxan vom Typ (a) ist, wobei die Gruppen R1 des Polysiloxans (b) mit den Gruppen R1 des Polysiloxans (a) reagieren können.

4. Zusammensetzung nach einem der Ansprüche 1 bis 3, **dadurch gekennzeichnet, dass** das Siliconcopolymer in Gegenwart eines Hydrosilylierungskatalysators durch Addition hergestellt wird von zumindest:
(a) einem alpha, omega-Divinylpolydimethylsiloxan und
(b) einem alpha, omega-Dihydrogenopolydimethylsiloxan.

5. Zusammensetzung nach einem der Ansprüche 1 bis 4, **dadurch gekennzeichnet, dass** das Siliconcopolymer in Form einer wässerigen Emulsion vorliegt.

6. Zusammensetzung nach einem der Ansprüche 1 bis 5, **dadurch gekennzeichnet, dass** das Siliconcopolymer in einer Konzentration von 0,05 bis 10 Gew.-%, bezogen auf das Gesamtgewicht der Zusammensetzung, enthalten ist.

7. Zusammensetzung nach einem der vorhergehenden Ansprüche, **dadurch gekennzeichnet, dass** das kationische Polymer ausgewählt ist unter:
(1) Homopolymeren oder Copolymeren, die von Acrylsäure- oder Methacrylsäureestern oder Acryl- oder Methacrylamiden abgeleitet sind und mindestens eine Einheit der folgenden Formeln enthalten: 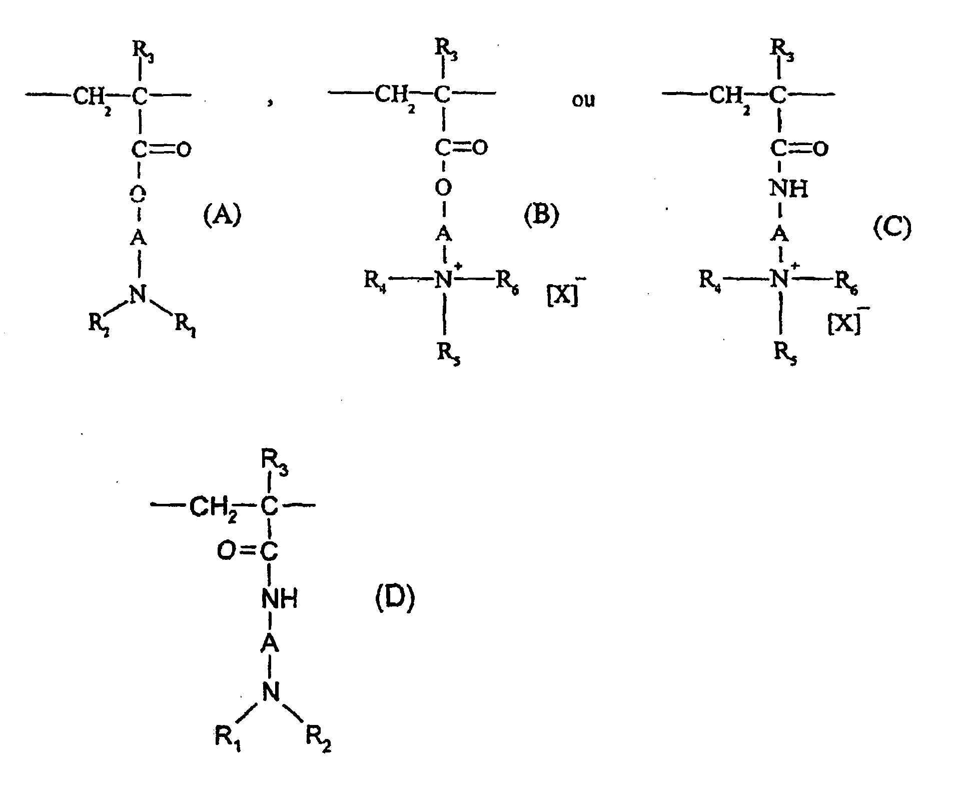 worin bedeuten:
die Gruppen R₃, die gleich oder verschieden sind, Wasserstoff oder die Gruppe CH₃;
die Gruppen A, die gleich oder verschieden sind, eine geradkettige oder verzweigte Alkylgruppe mit 1 bis 6 Kohlenstoffatomen oder eine Hydroxyalkylgruppe mit 1 bis 4 Kohlenstoffatomen;
die Gruppen R₄, R₅ und R₆, die identisch oder voneinander verschieden sind, eine Alkylgruppe mit 1 bis 18 Kohlenstoffatomen oder Benzyl;
die Gruppen R₁ und R₂, die gleich oder verschieden sind, Wasserstoff oder eine Alkylgruppe mit 1 bis 6 Kohlenstoffatomen; und
X ein Anion, das von einer anorganischen oder organischen Säure abgeleitet ist;
(2) Derivaten von Celluloseethern, die quartäre Ammoniumgruppen enthalten;
(3) Kationischen Cellulosederivaten, wie Cellulose-Copolymeren oder Cellulosederivaten, die mit einem wasserlöslichen quartären Ammoniummonomer gepfropft sind;
(4) Kationischen Polysacchariden;
(5) Polymeren, die aus Piperazinyl-Einheiten und zweiwertigen Alkylen- oder Hydroxyalkylen-Gruppen mit geraden oder verzweigten Ketten bestehen, die gegebenenfalls durch Sauerstoffatome, Schwefelatome oder Stickstoffatome oder aromatische oder heterocyclische Ringe unterbrochen sind, sowie den Oxidationsprodukten und/oder Quaternisierungsprodukten dieser Polymere;
(6) Wasserlöslichen Polyaminoamiden, die insbesondere durch Polykondensation einer Säure mit einem Polyamin hergestellt sind; diese Polyaminoamide können mit einem Epihalohydrin, einem Diepoxid, einem Dianhydrid, einem ungesättigten Dianhydrid, einem zweifach ungesättigten Derivat, einem Bis-halohydrin, einem Bis-azetidinium, einem Bis-haloacyldiamin, einem Alkyl-bis-halogenid oder auch einem Oligomer vernetzt sein, das bei der Umsetzung einer reaktiven bifunktionellen Verbindung und einem Bis-halohydrin, Bis-azetidinium, Bis-haloacyldiamin, Alkyl-bis-halogenid, Epihalohydrin, Diepoxid oder zweifach ungesättigten Derivat entsteht; das Vernetzungsmittel wird in Mengenanteilen von 0,025 bis 0,35 Mol pro Aminogruppe des Polyaminoamids eingesetzt. Die Polyaminoamide können alkyliert oder, wenn sie eine oder mehrere tertiäre Aminogruppen aufweisen, quaternisiert sein;
(7) Derivaten von Polyaminoamiden, die sich bei der Kondensation von Polyalkylenpolyaminen mit Polycarbonsäuren und anschließender Alkylierung mit bifunktionellen Mitteln entstehen;
(8) Polymeren, die durch Umsetzung eines Polyalkylenpolyamins, das zwei primäre Aminogruppen und mindestens eine sekundäre Aminogruppe aufweist, mit einer Dicarbonsäure hergestellt sind, die unter Diglykolsäure und gesättigten aliphatischen Dicarbonsäuren mit 3 bis 8 Kohlenstoffatomen ausgewählt ist;
(9) Alkyldiallylamin- oder Dialkyldiallylammonium-Cyclopolymeren;
(10) dem quartären Diammonium-Polymer mit wiederkehrenden Einheiten der folgenden Formel: 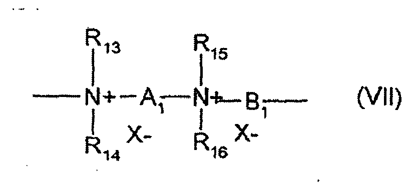 wobei in der Formel (VII) die Gruppen R₁₃, R₁₄, R₁₅ und R₁₆, die identisch oder voneinander verschieden sind, aliphatische, alicyclische oder arylaliphatische Gruppen mit 1 bis 20 Kohlenstoffatomen oder niedere hydroxyalkylaliphatische Gruppen bedeuten oder wobei die Gruppen R₁₃, R₁₄, R₁₅ und R₁₆ gemeinsam oder unabhängig voneinander mit den Stickstoffatomen, an die sie gebunden sind, Heterocyclen bilden, die gegebenenfalls ein zweites Heteroatom enthalten, das von Stickstoff verschieden ist, oder wobei die Gruppen R₁₃, R₁₄, R₁₅ und R₁₆ eine geradkettige oder verzweigte C₁₋₆₋Alkylgruppe bedeuten, die mit einer Nitril-, Ester-, Acyl- oder Amidgruppe oder -CO-O-R₁₇-D oder -CO-NH-R₁₇-D substituiert ist, worin R₁₇ eine Alkylengruppe und D eine quartäre Ammoniumgruppe bedeuten;
A₁ und B₁ bedeuten Polymethylengruppen mit 2 bis 20 Kohlenstoffatomen, die geradkettig oder verzweigt, gesättigt oder ungesättigt sein können und die an die Hauptkette gebunden oder in der Hauptkette einen oder mehrere aromatische Ringe oder ein oder mehrere Sauerstoffatome oder Schwefelatome oder eine oder mehrere der folgenden Gruppen enthalten können: Sulfoxid, Sulfon, Disulfid, Amino, Alkylamino, Hydroxy, quartäre Ammoniumgruppen, Ureido, Amid oder Ester;
X- bedeutet ein Anion, das von einer anorganischen oder organischen Säure abgeleitet ist;
Die Gruppen A₁, R₁₃ und R₁₅ können mit den beiden Stickstoffatomen, an die sie gebunden sind, einen Piperazinring bilden; wenn A₁ eine geradkettige oder verzweigte, gesättigte oder ungesättigte Alkylengruppe oder Hydroxyalkylengruppe bedeutet, kann die Gruppe B₁ auch eine Gruppe (CH₂)ₙ-CO-D-OC-(CH₂)ₙ- bedeuten, worin D bedeutet:
a) eine Glykolgruppe der Formel: -O-Z-O- , worin Z eine geradkettige oder verzweigte Kohlenwasserstoffgruppe oder eine Gruppe bedeutet, die einer der folgenden Formeln entspricht:
-(CH₂-CH₂-O)ₓ-CH₂-CH₂-
-[CH₂-CH(CH₃)-O]_{y}-CH₂-CH(CH₃)-
worin x und y eine ganze Zahl von 1 bis 4 bedeuten und einen wohl definierten und einzigen Polymerisationsgrad darstellen, oder eine beliebige Zahl von 1 bis 4 bedeuten und einen mittleren Polymerisationsgrad darstellen;
b) ein bis-sekundäres Diamin, beispielsweise ein Piperazinderivat,
c) ein bis-primäres Diamin der Formel : -NH-Y-NH- , worin Y eine geradkettige oder verzweigte Kohlenwasserstoffgruppe oder auch die zweiwertige Gruppe -CH₂-CH₂-S-S-CH₂-CH₂- bedeutet, oder
d) die Ureylengruppe der Formel : -NH-CO-NH-.
X- bedeutet vorzugsweise ein Anion;
(11) Quartären Polyammoniumpolymeren, die aus Einheiten der folgenden Formel (VIII) bestehen: 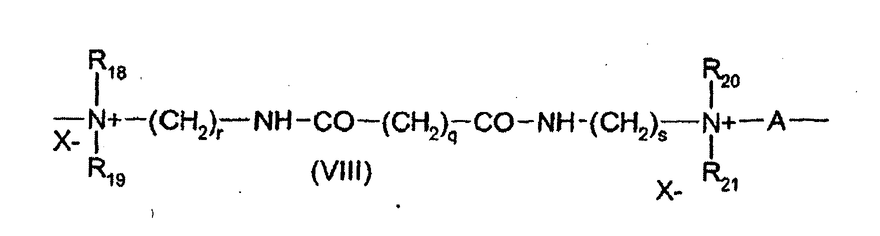 wobei in der Formel bedeuten:
R₁₈, R₁₉, R₂₀ und R₂₁, die identisch oder voneinander verschieden sind, ein Wasserstoffatom oder Methyl, Ethyl, Propyl, β-Hydroxyethyl, β-Hydroxypropyl oder -CH₂CH₂(OCH₂CH₂)ₚOH, wobei p Null oder eine ganze Zahl im Bereich von 1 bis 6 bedeutet, mit der Maßgabe, dass R₁₈, R₁₉, R₂₀ und R₂₁ nicht gleichzeitig Wasserstoff bedeuten,
r und s, die gleich oder verschieden sind, eine ganze Zahl im Bereich von 1 bis 6,
q Null oder eine ganze Zahl im Bereich von etwa 1 bis 34,
X ein Halogenatom, und
A ein Dihalogenid oder vorzugsweise -CH₂CH₂-O-CH₂CH₂-.
(12) Quartären Polymeren von Vinylpyrrolidon und Vinylimidazol;
(13) Polyaminen, die nach CTFA-Nomenklatur als 'POLYETHYLENGLYCOL (15) TALLOW POLYAMINE' bezeichnet werden;
(14) Vernetzten Polymeren von Methacryloyloxyalkyl(C₁₋₄)trialkyl(C₁₋₄)ammoniumsalzen;
(15) Polyalkyleniminen, insbesondere Polyethyleniminen, Polymeren, die Vinylpyridin- oder Vinylpyridinium-Einheiten enthalten, Kondensaten von Polyaminen und Epichlorhydrin, quartären Polyureylenen und Chitinderivaten.

8. Zusammensetzung nach dem vorhergehenden Anspruch, **dadurch gekennzeichnet, dass** das kationische Polymer unter den quartären Celluloseetherderivaten, Cyclopolymeren, kationischen Polysacchariden, Vinylpyrrolidon/ Methacrylamidopropyldimethylamin-Copolymeren, vernetzten Homopolymeren oder Copolymeren von Methacryloyloxyalkyl(C₁₋₄)trialkyl(C₁₋₄)ammoniumsalzen und deren Gemischen ausgewählt ist.

9. Zusammensetzung nach einem der vorhergehenden Ansprüche, **dadurch gekennzeichnet, dass** das kationische Polymer in einer Konzentration von 0,001 bis 20 Gew.-%, bezogen auf das Gesamtgewicht der Zusammensetzung, und vorzugsweise 0,01 bis 10 Gew.-%, enthalten ist.

10. Zusammensetzung nach einem der vorhergehenden Ansprüche, **dadurch gekennzeichnet, dass** sie ferner mindestens einen grenzflächenaktiven Stoff enthält, der unter den anionischen, nichtionischen, amphoteren oder kationischen grenzflächenaktiven Stoffen und deren Gemischen ausgewählt ist.

11. Zusammensetzung nach Anspruch 10, **dadurch gekennzeichnet, dass** der oder die grenzflächenaktive(n) Stoff(e) in einer Konzentration von 0,1 bis 60 Gew.-%, vorzugsweise 3 bis 40 Gew.-% und noch bevorzugter 5 bis 30 Gew.-%, bezogen auf das Gesamtgewicht der Zusammensetzung, enthalten sind.

12. Zusammensetzung nach einem der vorhergehenden Ansprüche, **dadurch gekennzeichnet, dass** sie ferner mindestens einen kationischen grenzflächenaktiven Stoff enthält.

13. Zusammensetzung nach dem vorhergehenden Anspruch, **dadurch gekennzeichnet, dass** der kationische grenzflächenaktive Stoff in Konzentrationen von 0,1 bis 10 Gew.-%, bezogen auf das Gesamtgewicht der Zusammensetzung, und vorzugsweise 0,5 bis 7 Gew.-% und noch bevorzugter 1 bis 5 Gew.-% enthalten ist.

14. Zusammensetzung nach einem der Ansprüche 1 bis 11 n **dadurch gekennzeichnet, dass** die Zusammensetzung mindestens einen Zusatzstoff enthält, der unter den Verdickungsmitteln, Parfums, Perlglanzstoffen, Konservierungsmitteln, Sonnenschutzfiltern, die gegebenenfalls siliconhaltig sind, Vitaminen, Provitaminen, amphoteren Polymeren, anionischen Polymeren oder nichtionischen Polymeren, Proteinen, Proteinhydrolysaten, 18-Methyleicosansäure, Hydroxysäuren, Panthenol, flüchtigen oder nicht flüchtigen, cyclischen oder linearen oder vernetzten, gegebenenfalls modifizierten Siliconen, Ceramiden, Pseudoceramiden, pflanzlichen Ölen, tierischen Ölen oder Mineralölen, synthetischen Ölen, pflanzlichen Ölen, tierischen Ölen, Mineralölen und synthetischen Ölen ausgewählt ist.

15. Zusammensetzung nach einem der vorhergehenden Ansprüche, **dadurch gekennzeichnet, dass** sie als Haarwaschmittel, Conditioner, Zusammensetzung für dauerhafte Verformungen, Entkräuselungen, Färbungen oder Entfärbungen der Haare, Zusammensetzung, die ausgespült wird und zwischen den beiden Schritten einer permanenten Verformung oder Entkräuselung aufgebracht wird, oder reinigende Zusammensetzung für den Körper vorliegt.

16. Verwendung einer Zusammensetzung nach einem der vorhergehenden Ansprüche zur Reinigung oder zur Pflege von Keratinsubstanzen.

17. Verfahren zur Behandlung von Keratinsubstanzen, beispielsweise zur Behandlung der Haare, das **dadurch gekennzeichnet ist, dass** es darin besteht, auf diese Substanzen eine kosmetische Zusammensetzung nach einem der Ansprüche 1 bis 15 aufzutragen und gegebenenfalls mit Wasser zu spülen.

18. Verwendung eines Siliconcopolymers nach einem der Ansprüche 1 bis 5 in einer kosmetischen Zusammensetzung, die ein kationisches Polymer enthält, oder zur Herstellung einer solchen Zusammensetzung.
